# FASCICULE DE BREVET EUROPEEN

(11) **EP 1 430 881 B1**
(45) Date de publication et mention de la délivrance du brevet: **27.09.2006**
(21) Numéro de dépôt: 03292859.0
(22) Date de dépôt: 18.11.2003
(51) Int. Cl.: A61K 8/49, A61Q 1/00, A61Q 1/02, A61Q 1/10, A61Q 3/02, A61Q 5/00, A61Q 5/02, A61Q 17/04, A61Q 19/00

(54) **Compositions contenant un dérivé de triazine solubilisé dans un mélange eutectique n-butylphthalimide/isopropylphthalimide, utilisations en cosmétique**
Zusammensetzungen enhaltend ein Triazinderivat, das in einem eutektischen Gemisch von n-Butylphthalimid/Isopropylphthalimid gelöst vorliegt, sowie Verwendung in kosmetischen Mitteln
Compositions comprising a triazine derivative solubilized in a eutectic mixture of n-butylphthalimide/isopropylphthalimide and cosmetic use

(30) Priorité: 16.12.2002 FR 0215910
(43) Date de publication de la demande: 23.06.2004
(73) Titulaire: L'ORÉAL, 75008 Paris (FR)
(72) Inventeur: Candau, Didier, 91570 Bievres (FR)
(74) Mandataire: Miszputen, Laurent

(56) Documents cités:
- EP-A- 0 850 935
- WO-A-00/66075
- WO-A-02/056851
- US-A- 5 489 431
- US-B1- 6 306 373
- "MAIN INGREDIENTS" BARE NECESSITIES, XX, XX, vol. 168, no. 2, février 2001 (2001-02), page 17 XP002950593

## Description

La présente invention concerne une composition photoprotectrice comprenant au moins un dérivé de 1,3,5 triazine solubilisé dans au moins un mélange eutectique de n-butylphthalimide et d'isopropylphthalimide ainsi que ses diverses utilisations cosmétiques notamment la protection de la peau et/ou des lèvres et/ou des phanères contre le rayonnement ultra-violet, en particulier le rayonnement solaire.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions anti-solaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection solaire recherché, le facteur de protection solaire (FPS) s'exprimant mathématiquement par le rapport de la dose de rayonnement UV nécessaire pour atteindre le seuil érythématogène avec le filtre UV sur la dose de rayonnement UV nécessaire pour atteindre le seuil érythématogène sans filtre UV.

Les dérivés de 1,3,5-triazine sont particulièrement recherchés dans la cosmétique solaire du fait qu'ils sont fortement actifs dans l'UVB, et même dans l'UV-A pour certains de ces composés selon la nature des substituants mis en jeu. De plus, ils sont photostables, c'est à dire qu'ils se dégradent peu ou pas chimiquement sous l'action du rayonnement UV. Ils sont notamment décrits dans les demandes de brevet US 4367390, EP863145, EP517104, EP570838, EP796851, EP775698, EP878469 et EP933376, et on connaît en particulier :
- la 2,4,6-tris[p-(2'-éthylhexyl-1'-oxycarbonyl)anilino]-1,3,5-triazine ou « Ethylhexyl Triazone » (nom INCI), vendue sous la dénomination commerciale « Uvinul T 150 » par la société BASF,
- la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » (nom INCI) vendu sous le nom commercial « UVASORB HEB » par SIGMA 3V,
- la 2,4-bis {[4-2-éthyl-hexyloxy)]-2-hydroxy]-phenyl}-6-(4-méthoxy-phenyl)-1,3,5-triazine ou « Anisotriazine » (nom INCI) vendu sous le nom commercial «TINOSORB S » par CIBA SPECIALTY CHEMICALS.

Dans l'art antérieur, il a été proposé d'utiliser des dérivés de 1,3,5-triazine dans des huiles comme des esters et plus particulièrement des benzoates d'alkyles en C12-C15 ("FINSOLV TN" de chez Finetex), ou des triglycérides et notamment des triglycérides d'acides gras en C8-C12 ("MIGLYOL 812" de chez Hüls), ou encore des monoalcools ou des polyols gras oxyéthylénés ou oxypropylénés ( « CETIOL HE » de chez Henkel ou « WITCONOL AM» de chez WITCO ). L'utilisation de ces huiles présentent deux inconvénients:
- soit l'apparition au cours du temps, de cristallisation dans les formules qui nuisent aux qualités cosmétiques, à la stabilité et à l'efficacité des produits solaires ;
- soit la limitation de la concentration en filtres dans les formules ce qui ne permet pas d'obtenir des produits suffisamment efficaces.

Le problème technique posé à la base de la présente invention est donc d'améliorer l'efficacité photoprotectrice, les propriétés cosmétiques et la stabilité des compositions contenant de tels dérivés de 1,3,5-triazine.

De manière inattendue et surprenante, la demanderesse a découvert que l'on pouvait résoudre le problème technique évoqué ci-dessus en ajoutant un mélange eutectique de n-butyl- et d'isopropylphthalimide dans une composition contenant au moins un dérivé de triazine, dans un quantité suffisante permettant de solubiliser à lui seul la quantité totale de dérivé de triazine.

Ce mélange eutectique de n-butylphthalimide et d'isopropylphthalimide permet l'obtention de compositions anti-solaires stables contenant des dérivés 1,3,5 triazine et qui présentent un facteur de protection solaire supérieur à ceux des compositions de l'art antérieur contenant dérivés 1,3,5 triazine. Ces compositions possèdent en outre des qualités cosmétiques améliorées. Elles permettent notamment une bonne hydratation de la peau c'est-à-dire que l'on n'observe pas de dessèchement de la peau, ni un toucher trop gras.

La présente invention a donc pour objet une composition photoprotectrice contenant au moins un dérivé de triazine et au moins un mélange eutectique de n-butylphthalimide et d'isopropylphthalimide dans une quantité suffisante permettant de solubiliser à lui seul la quantité totale de dérivé de triazine.

Un autre objet de l'invention consiste en l'utilisation d'une telle composition pour la fabrication de compositions cosmétiques ou dermatologiques en particulier destinées à la protection des matières kératiniques contre le rayonnement solaire.

L'invention a également pour objet l'utilisation d'au moins un mélange eutectique n-butylphthalimide/isopropylphthalimide dans une composition cosmétique ou dermatologique photoprotectrice contenant au moins un dérivé de 1,3,5-triazine en vue d'améliorer le facteur de protection solaire et/ou les qualités cosmétiques et/ou la stabilité de cette composition.

D'autres objets de l'invention apparaîtront à la lecture de la description et des exemples qui suivent.

On entend par composition photoprotectrice, toute composition contenant au moins un composé organique et/ou au moins composé minéral susceptible de filtrer les radiations UV-A et/ou UV-B par phénomène d'absorption, réflexion ou de diffusion.

Le mélange eutectique conforme à l'invention est de préférence constitué de :
(a) 60 à 75 % en poids de n-butyphthalimide de structure (1) suivante : 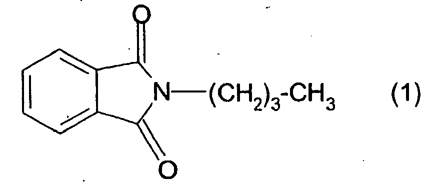
(b) 25 à 40% en poids d'isopropylphthalimide conforme à invention correspond à la structure suivante :

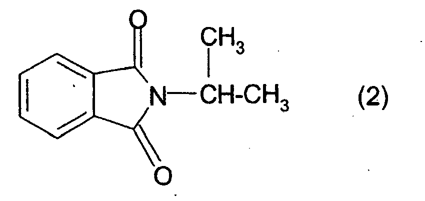

Les mélanges eutectiques n-butylphthalimide/isopropylphthalimide conformes à l'invention sont connus et sont décrits et synthétisés dans le brevet US 6,306,373 (faisant partie intégrante du contenu de la description).

Parmi les mélanges eutectiques utilisables, on peut citer les mélanges suivants :
n-butylphthalimide/isopropylphthalimide (60/40 % en poids)
n-butylphthalimide/isopropylphthalimide (62/38 % en poids)
n-butylphthalimide/isopropylphthalimide (65/35 % en poids)
n-butylphthalimide/isopropylphthalimide (70/30 % en poids)
n-butylphthalimide/isopropylphthalimide (75/25 % en poids)

On utilisera par exemple le mélange eutectique n-butylphthalimide/isopropylphthalimide commercialisé sous le nom PELEMOL BIP par la société Phoenix Chemicals.

Le mélange eutectique conforme à l'invention sera de préférence utilisé à des concentrations allant de 0,1 à 50% en poids et plus préférentiellement de 1 à 30 % en poids par rapport au poids total de la composition.

Le dérivé de 1,3,5-triazine répond à la formule (I) suivante : 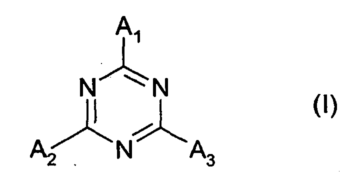 dans laquelle les radicaux A1, A2 et A3, identiques ou différents sont choisis parmi les groupes de formules (II) à (IX) suivantes : 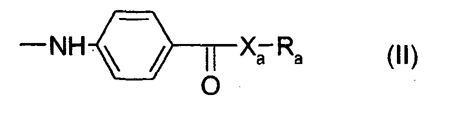 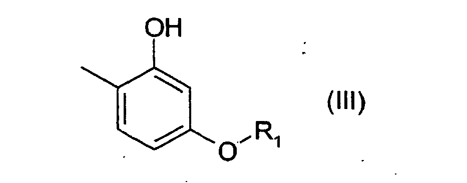 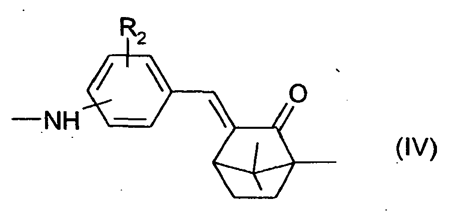 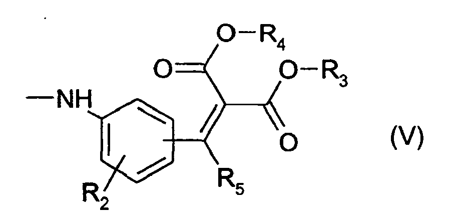 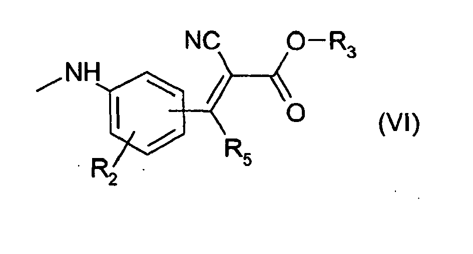 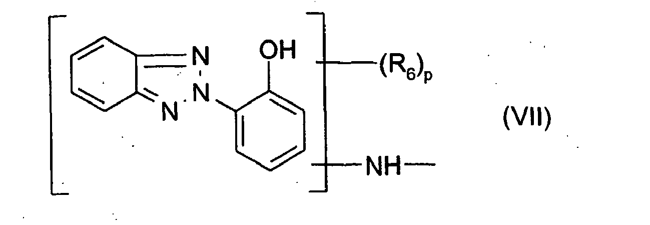 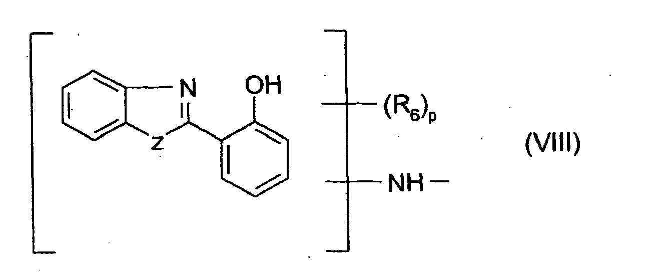 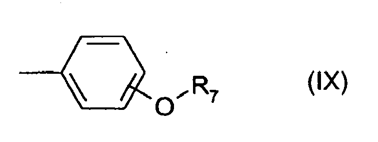 dans lesquelles :
- Xₐ (chacun des Xa peut être identique ou différent) représente l'oxygène ou -NH-;
- Rₐ (chacun des Rₐ peut être identique ou différent) est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C₁₈, de préférence en C₆-C₁₂; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 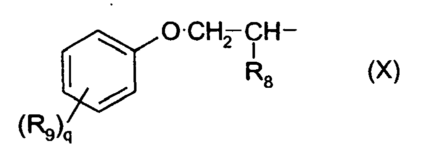 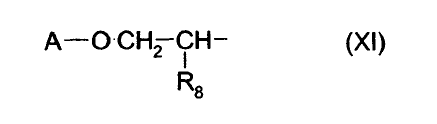 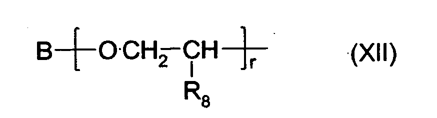
dans lesquelles :
- R₈ est l'hydrogène ou un radical méthyle;
- R₉ est un radical alkyle en C₁-C₉;
- q est un nombre entier égal à 0; 1; 2; 3;
- r est un nombre entier égal à 1; 2; 3; 4; 5; 6; 7; 8; 9; 10;
- A est un radical alkyle en C₄-C₈ ou un radical cycloalkyle en C₅-C₈ ;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.
- R₁ désigne un radical alkyle en C₃-C₁₈ ; un radical alcényle en C₂-C₁₈ ; un reste de formule -CH₂-CH(OH)-CH₂-OT₁ où T₁ est un atome d'hydrogène ou un radical alkyle en C₁-C₈ ; un reste de formule (XIII) suivante : 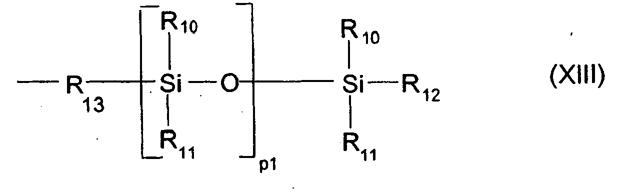
dans laquelle :
- R₁₃ désigne une liaison covalente ; un radical alkyle linéaire ou ramifié en C₁-C₄ ou bien un radical de formule -Cₘ₁H₂ₘ₁-O- où m₁ est un entier égal à 1; 2; 3; 4 ;
- p₁ est un entier égal à 0; 1; 2; 3; 4; 5 ;
- les radicaux R₁₀, R₁₁ et R₁₂, identiques ou différents, désignent un radical alkyle en C₁₋C₁₈ ; un radical alcoxy en C₁-C₁₈ ou un radical de formule : 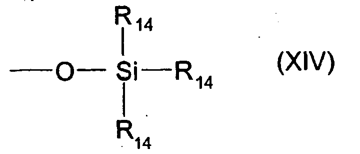
où R₁₄ est un radical alkyle en C₁-C₅.
- R₂ désigne un atome d'hydrogène, un radical alkyle en C₁-C₄ linéaire ou ramifié ou un radical alkoxy en C₁-C₄ ;
- R₃ et R₄ , identiques ou différents désignent un radical alkyle linéaire ou ramifié en C₁₋C₂₀ ;
- R₅ représente un atome d'hydrogène ou un radical phényle éventuellement substitué par un halogène ou par un radical alkyle en C₁-_{C4} ou par un radical alcoxy en C₁-C₄ ;
- R₆ est un radical alkyle en C₁-C₈ linéaire ou ramifié, alkoxy en C₁-C₃ étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₇ désigne un atome d'hydrogène, un radical alkyle en C₁-C₁₀, un radical de formule : -(CH₂CHR₅-O)ₙ₁R8 où n₁ est un nombre de 1 à 16, ou bien un radical de structure -CH₂-CH-(OH)-CH₂OT₁ avec R₈ et T₁ ayant la même signification indiquée ci-dessus. - Z représente l'oxygène, le soufre, -NH- ou -NR₃ - avec R₃ représentant un radical alkyle en C₁-C₂₀, linéaire ou ramifié;
- p est 0, 1, 2 ou 3,
A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

Une première famille plus particulièrement préférée de dérivés de 1,3,5-triazine, notamment décrite dans le document EP-A-0 517 104, est celle des 1,3,5-triazines répondant à la formule (I) dans laquelle les A1, A2 et A3 sont de formule (II) et présentant l'ensemble des caractéristiques suivantes :
- l'un des groupements Xₐ-Rₐ représente le radical -NH-Rₐ avec Ra choisi parmi : un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄ ;
- R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

Une deuxième famille plus particulièrement préférée de dérivés de 1,3,5-triazine, notamment décrite dans le document EP-A-0 570 838, est celle des 1,3,5-triazines répondant à la formule (I) dans laquelle les A₁, A₂ et A₃ sont de formule (II) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄ ; un radical de formule (X), (XI) ou (XII) ci-dessus dans lesquelles B est un radical alkyle en C₁-C₄ et R₉ est le radical méthyle ;
- le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles B est un radical alkyle en C₁-C₄ et R₉ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 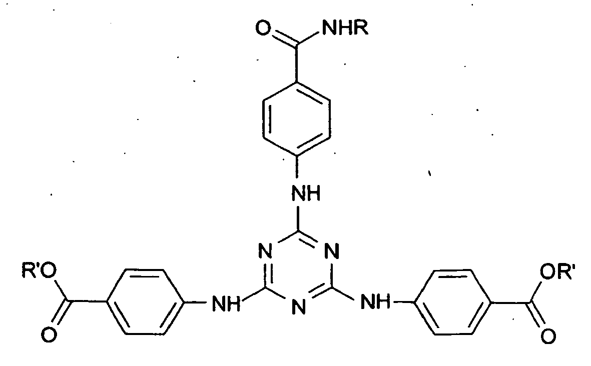 dans laquelle R' désigne un radical éthyl-2 hexyle et R désigne un radical tert-butyle.

Une troisième famille préférée de composés utilisables dans le cadre de la présente invention, et qui est notamment décrite dans le document US 4,724,137, est celle des 1,3,5-triazines répondant à la formule (I) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 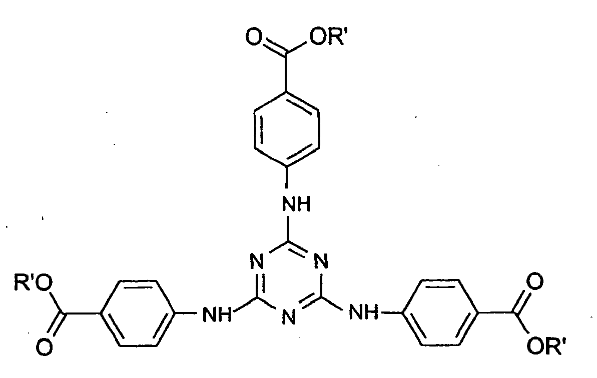 dans laquelle R' désigne un radical 2-éthyl hexyle.

Une quatrième famille préférée de composés utilisables dans le cadre de la présente invention, et qui est notamment décrite dans la demande de brevet EP-A-0775698 est celle des 1,3,5-triazines répondant à la formule (I) dans laquelle les A₁ et A₂ sont de formule (III) et A₃ est de formule (IX) et présentant l'ensemble des caractéristiques suivantes : R₁, identiques ou différents, désignent un radical alkyle en C₃-C₁₈ ; un radical alcényle en C₂-C₁₈ ou bien un reste de formule -CH₂-CH(OH)-CH₂-OT₁ où T₁ est un atome d'hydrogène ou un radical alkyle en C₁-C₈ ; R₇ désigne un atome d'hydrogène, un radical alkyle en C₁-C₁₀.

Une 1,3,5-triazine particulièrement préférée de cette quatrième famille est la 2,4-bis {[4-2-éthyl-hexyloxy)]-2-hydroxy]-phenyl}-6-(4-méthoxy-phenyl)-1,3,5-triazine ou « Anisotriazine » vendue sous le nom commercial «TINOSORB S» par CIBA SPECIALTY CHEMICALS et répond à la formule suivante : 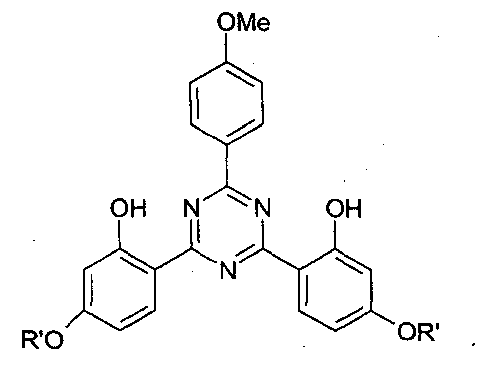 dans laquelle R' désigne un radical éthyl-2 hexyle.

Une cinquième famille préférée de composés utilisables dans le cadre de la présente invention, et qui est notamment décrite dans les demandes de brevet EP507691, EP507692, EP790243 et EP944624 et dont le contenu technique est intégré totalement à la présente description est celle des 1,3,5-triazines répondant à la formule (I) dans laquelle les A1, A2 et A3 sont de formules (VII) à (XI) citées précédemment.

A titre d'exemples de ces composés de formule utilisables, on peut citer :
- la 2,4,6-tris-(4'-amino benzalmalonate de diisobutyle)-s-triazine,
   la 2,4,6-tris-(4'-amino benzalmalonate de di(2-éthylhexyle))-s-triazine,
   la 2,4,6-tris-(4'-amino benzalmalonate de di(2-éthylhexyle))-6-chloro-s-triazine,
   la 2,4,6-tris-(4'-amino benzalmalonate de di(2-éthylhexyle))-6-(4'-aminobenzoate de 2-éthylhexyle)-s-triazine,
   la 2,4,6-tris-(4'-amino benzalmalonate de diisobutyle)-6-butoxy-s-triazine,
   la 2,4,6-tris-(4'-amino benzalmalonate de diisobutyle)-6-(2-éthylhexylamino)-s-triazine,
   la 2,4-bis-(4'-aminobenzylidène camphre)-6-(2-éthylhexyl-amino)-s-triazine,
   la 2,4-bis-(4'-aminobenzylidène camphre)-6-(4'-amino benzalmalonate de diisobutyle)-s-triazine,
   la 2,4,6-tris(4'-amino benzalmalonate de diéthyle)-s-triazine,
   la 2,4,6-tris(4'-amino benzalmalonate de diisopropyle)-s-triazine,
   la 2,4,6-tris(4'-amino benzalmalonate de diméthyle)-s-triazine,
   la 2,4,6-tris(α-cyano-4-aminocinnamate d'éthyle)-s-triazine,
   la 2,4,6-tris[(3'-benzotriazol-2-yl-2'-hydroxy-5'-methyl) phenylamino]-s-triazine,
   2,4,6-tris[(3'-benzotriazol-2-yl-2'-hydroxy-5'-ter-octyl) phénylamino]-s-triazine.

Une 1,3,5-triazine particulièrement préférée de cette cinquième famille est la 2,4,6-tris-(4'-amino benzalmalonate de diisobutyle)-s- triazine qui répond à la formule suivante :

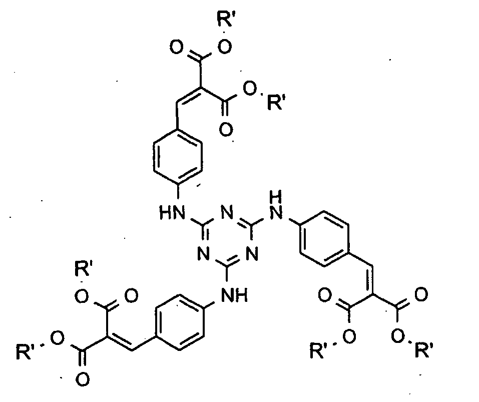

Les compositions selon la présente invention comprennent de préférence, dans un milieu physiologiquement acceptable de 0,05 à 15%, de préférence de 0,1 à 10 % de dérivés 1,3,5 triazine en poids par rapport au poids total de ladite composition.

Ladite composition selon la présente invention est de préférence une composition cosmétique contenant outre le dérivé 1,3,5 triazine en tant que filtre organique, au moins un autre filtre organique et/ou au moins un autre filtre minéral complémentaire hydrosoluble, liposoluble ou insoluble dans les solvants cosmétiques couramment utilisés.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

### Dérivés du dibenzoylméthane :

Butyl Methoxydibenzoylmethane vendu notamment sous le nom commercial « PARSOL 1789 » par HOFFMANN LA ROCHE,
lsopropyl Dibenzoylmethane,

### Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

### Dérivés de β,β-diphénylacrylate:

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK ,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP »par Haarmann et REIMER,

### Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Dérivés du benzalmalonate :

Polyorganosiloxane à fonctions benzalmalonate comme le Polysilicone-15 vendu sous la dénomination commerciale « PARSOL SLX » par. HOFFMANN LA ROCHE

### Dérivés de 4,4-diarylbutadiène :

- 1,1-dicarboxy(2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3, 5-triazine vendu sous le nom d'Uvasorb K2A par Sigma 3V
et leurs mélanges.

Les filtres UV organiques complémentaires préférentiels sont choisis parmi
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethykhexyl)-imino-1,3,5-triazine
et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les, insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans lé domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C10-C30alkylacrylates du type Pemulen ou le polyacrylate-3 vendu sous le nom VISCOPHOBE DB 1000 par Amerchol ; les polyacrylamides tels que l'émulsion polyacrylamide, C13-C14 isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

La présente invention concerne également l'utilisation dudit mélange eutectique n-butylphthalimide/isopropylphthalimide dans une composition cosmétique ou dermatologique contenant un dérivé de 1,3,5-triazine comme solvant dudit dérivé de triazine dans ladite composition.

La présente invention concerne également l'utilisation dudit mélange eutectique n-butylphthalimide/isopropylphthalimide dans une composition cosmétique ou dermatologique contenant un dérivé de 1,3,5-triazine en vue d'améliorer le facteur de protection solaire, les propriétés cosmétiques et/ou la stabilité de cette composition.

Des exemples concrets, mais nullement limitatifs, illustrant l'invention, vont maintenant être donnés.

### Etude comparative de la solubilité d'un dérivé de triazine dans différents solvants

On a déterminé la solubilité d'un dérivé de triazine (ie : 2,4,6-tris-(4'-amino benzalmalonate de diisobutyle)-s-triazine) dans différents solvants au moyen du test suivant :
- à température ambiante, on ajoute 20 g de solvant à 5 g de filtre,
- on porte le mélange ainsi obtenu à 83° C au moyen d'un bain-marie,
- on observe la présence ou non de cristaux après 24 heures et retour à température ambiante :

Les résultats obtenus sont rassemblés dans le tableau ci-dessus (les noms chimiques sont donnés dans la nomenclature CTFA, 5ème édition, 1993).

| **SOLVANT** | **SOLUBILITE DU FILTRE TRIAZINE** |
|---|---|
| **mélange eutectique n-butylphthalimide/isopropylphthalimide** | **40%** |
| **PELEMOL BIP de chez Phoenix Chemicals (invention)** | |
| Triglycérides d'acides gras en C₈-C₁₂ | < 10% |
| "MIGLYOL 812" de chez Hüls | |
| PPG-3 Myristyl Ether | <10% |
| "Witconol APM" de chez Witco | |
| Benzoate d'alcools en C₁₂-C₁₅ | < 10% |
| "FINSOLV TN" de chez Finetex | |

Ces résultats démontrent clairement le pouvoir solubilisant supérieur du mélange eutectique de n-butylphthalimide/isopropyle phthalimide conforme à l'invention et l'effet bénéfique remarquable apporté par la présence de l'huile au niveau de la solubilité dans les compositions finales.

### Test d'efficacité in vitro :

La méthode d'évaluation du facteur de protection utilisée est la méthode in vitro décrite par B. L. DIFFEY et al. dans J. Soc. Cosmet. Chem. 40-127-133 (1989), qui consiste à déterminer les facteurs de protection monochromatiques tous les 5 nm dans une gamme de longueurs d'onde de 290 à 400nm, et à calculer, à partir de ceux-ci, le facteur de protection solaire selon une équation mathématique donnée.

### Résultats :

On a formulé dans un support de type H/E, à 5% en poids, deux filtres de type triazine dans le solvant selon l'invention et dans un solvant de comparaison (Finsolv TN) et déterminé pour chaque formule, trois valeurs de SPF, dont la moyenne et l'écart type sont regroupés dans le tableau suivant :

| **COMPOSITION** | **Quantité** |
|---|---|
| Mélange d'alcool cétylstéarylique et d'alcool cétylstéarylique oxyéthyléné (33 OE) 80/20 | 7 |
| (SINNOWAX AO -HENKEL) | |
| Mélange de mono et distéarate de glycérol (CERASYNT SD-V ISP) | 2 |
| Alcool cétylique | 1.5 |
| Polydiméthyl siloxane (DOW CORNING 200 FLUID -DOW CORNING) | 1.5 |
| **SOLVANT** | **15** |
| **FILTRE DE TYPE TRIAZINE** | **5** |
| glycérine | 20 |
| triéthanolamine | qs pH 7 |
| conservateurs | qs |
| Eau déminéralisée qsp | 100 g |

| **Filtres** | **Solvants** | **SPF moyen** |
|---|---|---|
| **(5%)** | **(15%)** | **(écart-type)** |
| 2,4,6-tris-(4'-amino benzalmalonate de diisobutyle)-s-triazine | n-butylphthalimide/isopropylphthalimide PELEMOL BIP de chez Phoenix Chemicals (invention) | 8.0 |
| | | (0.7) |
| | Benzoate d'alcools en C₁₂-C₁₅ | 4.1 |
| | "FINSOLV TN" de chez Finetex | (0.1) |
| 2,4-bis {[4-2-éthyl-hexyloxy)]-2-hydroxy]-phenyl}-6-(4-méthoxy-phenyl)-1,3,5-triazine | n-butylphthalimide/isopropyle phthalimide PELEMOL BIP de chez Phoenix Chemicals (invention) | 11.4 |
| | | (2) |
| « Anisotriazine » vendue sous le nom commercial «TINOSORB S » par CIBA SPECIALTY CHEMICALS | Benzoate d'alcools en C₁₂-C₁₅ | 8.5 |
| | "FINSOLV TN" de chez Finetex | (1.4) |

Pour le même système photoprotecteur, l'émulsion selon l'invention contenant comme solvant le mélange eutectique n-butylphthalimide/isopropyle phthalimide présente un SPF significativement plus élevé.

## Revendications

1. Composition photoprotectrice, **caractérisée par le fait qu'**elle comprend,
(i) au moins un dérivé de 1,3,5-triazine, et
(ii) au moins un mélange eutectique constitué de :
(a) n-butyphthalimide de structure (1) suivante : 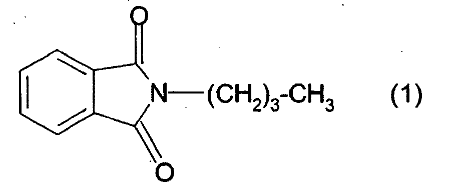
(b) d'isopropylphthalimide de structure (2) suivante : 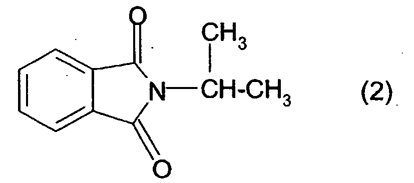
dans un quantité suffisante permettant de solubiliser à lui seul la quantité totale de dérivé de triazine.

2. Composition selon la revendication 1 où ledit mélange eutectique contient de 60 à 75% en poids de n-butylphthalimide

3. Composition selon la revendication 1 ou 2, où le mélange eutectique est choisi parmi les mélanges suivants :
n-butylphthalimide/isopropylphthalimide (60/40 % en poids)
n-butylphthalimide/isopropylphthalimide (62/38 % en poids)
n-butylphthalimide/isopropylphthalimide (65/35 % en poids)
n-butylphthalimide/isopropylphthalimide (70/30 % en poids)
n-butylphthalimide/isopropylphthalimide (75/25 % en poids)

4. Composition selon l'une quelconque des revendications 1 à 3, où le mélange eutectique est présent à des concentrations allant de 0,1 à 50% en poids et plus préférentiellement de 1 à 30 % en poids par rapport au poids total de la composition.

5. Composition selon l'une quelconque des revendications 1 à 4, où le dérivé de 1,3,5-triazine répond à la formule (I) suivante : 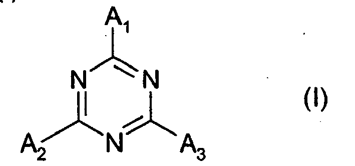 dans laquelle les radicaux A₁, A₂ et A₃, identiques ou différents sont choisis parmi les groupes de formules (II) à (IX) suivantes : 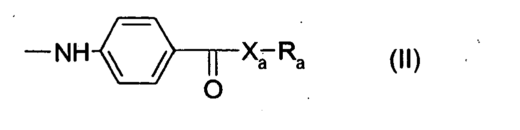 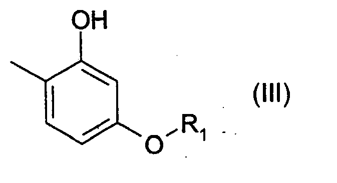 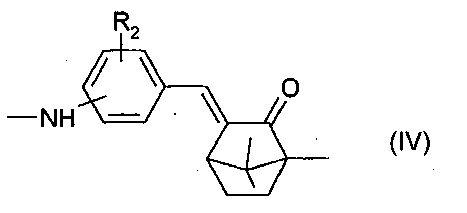 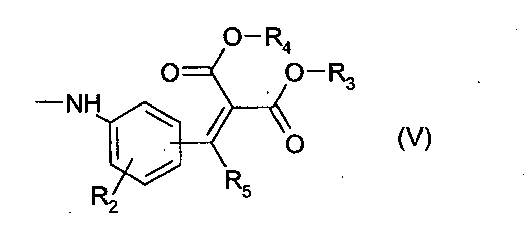 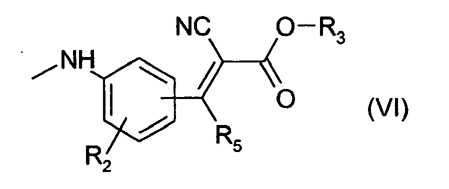 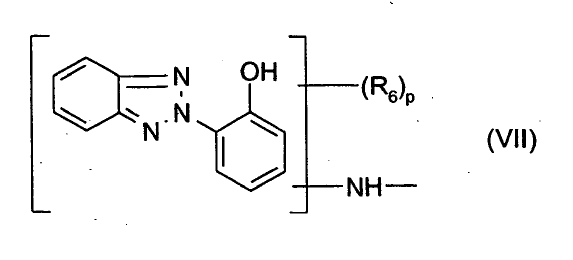 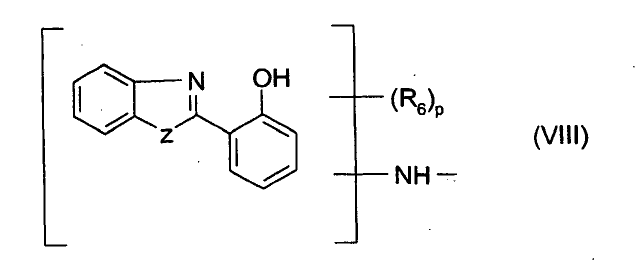 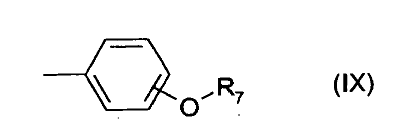 dans lesquelles :
- Xₐ (chacun des Xₐ peut être identique ou différent) représente l'oxygène ou -NH-;
- Rₐ (chacun des Ra peut être identique ou différent) est choisi parmi l'hydrogène; un métal alcalin; un radical ammonium éventuellement substitué par un ou plusieurs radicaux alkyles linéaire ou ramifié en C₁-C₁₈ ou hydroxyalkyles linéaire ou ramifié en C₁-C₁₈; un radical alkyle linéaire ou ramifié en C₁-C₁₈, de préférence en C₆-C₁₂; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (X), (XI) ou (XII) suivantes : 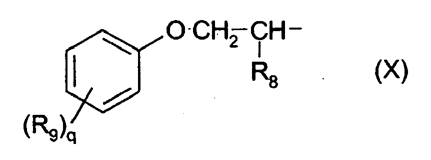 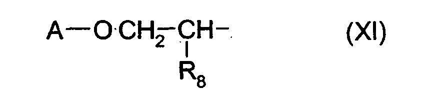 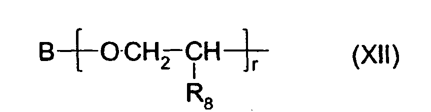
dans lesquelles :
- R₈ est l'hydrogène ou un radical méthyle;
- R₉ est un radical alkyle en C1-C9;
- q est un nombre entier égal à 0; 1; 2; 3;
- r est un nombre entier égal à 1; 2; 3; 4; 5; 6; 7; 8; 9; 10;
- A est un radical alkyle en C₄-C₈ ou un radical cycloalkyle en C₅-C₈;
- B est choisi parmi : un radical alkyle linéaire ou ramifié en C1-C8; un radical cycloalkyle en C₅-C₈; un radical aryle éventuellement substitué par un ou plusieurs radicaux alkyles en C₁-C₄.
- R₁ désigne un radical alkyle en C₃-C₁₈; un radical alcényle en C₂-C₁₈ ; un reste de formule -CH₂-CH(OH)-CH₂-OT₁ où T₁ est un atome d'hydrogène ou un radical alkyle en C₁-C₈; un reste de formule (XIII) suivante : 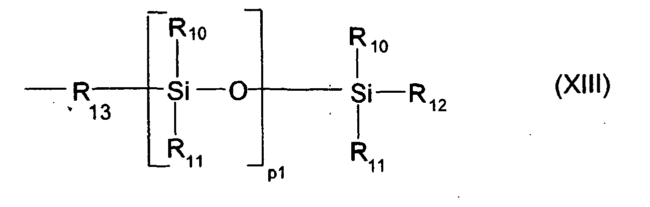
dans laquelle :
- R₁₃ désigne une liaison covalente ; un radical alkyle linéaire ou ramifié en C₁-C₄ ou bien un radical de formule -Cₘ₁H₂ₘ₁-O- où m₁ est un entier égal à 1; 2; 3; 4 ;
- p₁ est un entier égal à 0; 1; 2; 3; 4; 5 ;
- les radicaux R₁₀, R₁₁ et R₁₂, identiques ou différents, désignent un radical alkyle en C₁₋C₁₈ ; un radical alcoxy en C₁-C₁₈ ou un radical de formule : 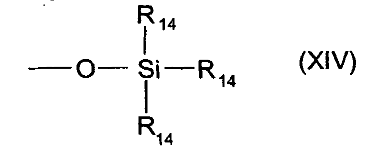
où R₁₄ est un radical alkyle en C₁-C₅.
- R₂ désigne un atome d'hydrogène, un radical alkyle en C1-C4 linéaire ou ramifié ou un radical alkoxy en C1-C4 ;
- R₃ et R₄, identiques ou différents désignent un radical alkyle linéaire ou ramifié en C₁₋C₂₀ ;
- R₅ représente un atome d'hydrogène ou un radical phényle éventuellement substitué par un halogène ou par un radical alkyle en C₁-C₄ ou par un radical alcoxy en C₁-C₄ ;
- R₆ est un radical alkyle en C₁-C₈ linéaire ou ramifié, alkoxy en C1-C3 étant entendu que, dans ce dernier cas, deux R₆ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₇ désigne un atome d'hydrogène, un radical alkyle en C₁-C₁₀, un radical de formule : -(CH₂CHR₅-O)ₙ1R₈ où n₁ est un nombre de 1 à 16, ou bien un radical de structure -CH₂-CH-(OH)-CH₂OT₁ avec R₈ et T₁ ayant la même signification indiquée ci-dessus.
- Z représente l'oxygène, le soufre, -NH- ou -NR₃ - avec R₃ représentant un radical alkyle en C₁-C₂0 linéaire ou ramifié;
- p est 0, 1, 2 ou 3,
A₁ peut également être un halogène, un radical -N(R₃)₂, les deux R₃ pouvant former ensemble un cycle de 4 ou 5 atomes de carbone, ou un groupe -OR₃, R₃ ayant la même définition qu'au dessus.

6. Composition selon la revendication 5, où le dérivé de 1,3,5-triazine répond à la formule
(I) dans laquelle les A₁, A₂ et A₃ sont de formule (II) et présentant l'ensemble des caractéristiques suivantes :
- l'un des groupements Xₐ-Rₐ représente le radical -NH-Rₐ avec Rₐ choisi parmi : un radical cycloalkyle en C₅-C₁₂ éventuellement substitué par un ou plusieurs radicaux alkyles en C1-C4; un radical de formule (X), (XI) ou (XII) ci-dessus dans lesquelles :
- B est un radical alkyle en C₁-C₄ ;
- R₉ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄ ; un radical de formule (X), (XI) ou (XII) ci-dessus dans lesquelles :
- B est un radical alkyle en C₁-C₄;
- R₉ est le radical méthyle.

7. Composition selon la revendication 5, où le dérivé de 1,3,5-triazine répond à la formule
(I) dans laquelle les A₁, A₂ et A₃ sont de formule (II) et présentant l'ensemble des caractéristiques suivantes :
- un ou deux Xₐ-Rₐ représente le radical -NH-Rₐ, avec Ra choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (X), (XI) ou (XII) ci-dessus dans lesquelles :
- B est un radical alkyle en C₁-C₄ ;
- R9 est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (X), (XI) ou (XII) ci-dessus dans lesquelles :
- B est un radical alkyle en C₁-C₄;
- R₉ est le radical méthyle.

8. Composition selon la revendication 7, où le dérivé de 1,3,5-triazine est la 2-[(p-(tertiobutylamido)anilino]-4,6-bis-[(p-(2'-éthylhexyl-1'-oxycarbonyl)anilino]-1,3,5-triazine de formule suivante : 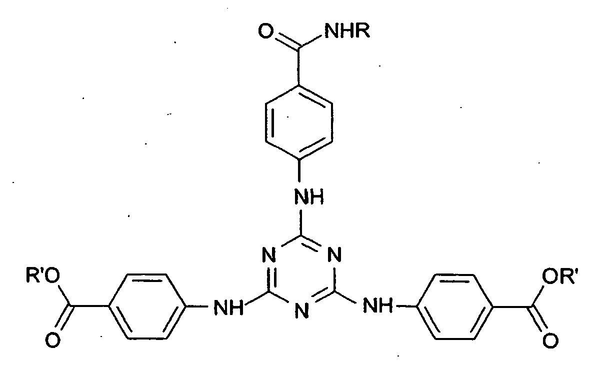 dans laquelle R' désigne un radical éthyl-2 hexyle et R désigne un radical tert-butyle.

9. Composition selon la revendication 5, où le dérivé de 1,3,5-triazine répond à la formule
(I) dans laquelle les A₁, A₂ et A₃ sont de formule (II) et présentant l'ensemble des caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

10. Composition selon la revendication 9, où le dérivé de 1,3,5-triazine est la 2,4,6-tris[p-(2'-éthylhexyl-1'-oxycarbonyl)anilino]-1,3,5-triazine de formule suivante : 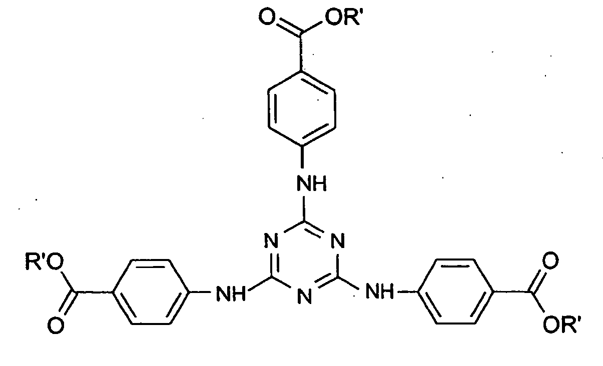 dans laquelle R' désigne un radical 2-éthyl hexyle.

11. Composition selon la revendication 5, où le dérivé de 1,3,5-triazine où le dérivé de 1,3,5-triazine répond à la formule (I) dans laquelle les A₁ et A₂ sont de formule (III) et A₃ est de formule (IX) et présentant l'ensemble des caractéristiques suivantes : R1, identiques ou différents, désignent un radical alkyle en (C₃-C₁₈, un radical alcényle en C₂-C₁₈ ou bien un reste de formule -CH₂-CH(OH)-CH₂-OT₁ où T₁ est un atome d'hydrogène ou un radical alkyle en C₁-C₈ ; R₇ désigne un atome d'hydrogène, un radical alkyle en C₁-C₁₀.

12. Composition selon la revendication 11, où le dérivé de 1,3,5-triazine est la 2,4-bis {[4-2-éthyl-hexyloxy)]-2-hydroxy]-phenyl}-6-(4-méthoxy-phenyl)-1,3,5-triazine de formule suivante : 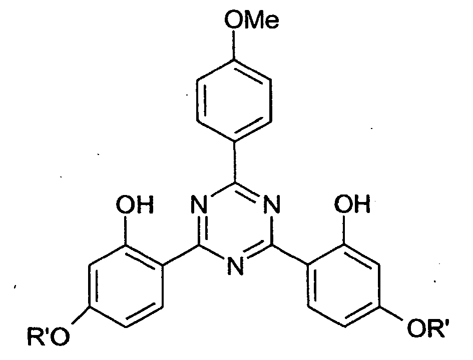 dans laquelle R' désigne un radical éthyl-2 hexyle.

13. Composition selon la revendication 5, où le dérivé de 1,3,5-triazine répond à la formule (I) dans laquelle dans laquelle les A₁, A₂ et A₃ sont de formules (VII) à (XI).

14. Composition selon la revendication 13, où le dérivé de 1,3,5-triazine est choisi parmi :
- la 2,4,6-tris-(4'-amino benzalmalonate de diisobutyle)-s-triazine,
la 2,4,6-tris-(4'-amino benzalmalonate de di(2-éthylhexyle))-s-triazine,
la 2,4,6-tris-(4'-amino benzalmalonate de di(2-éthylhexyle))-6-chloro-s-triazine,
la 2,4,6-tris-(4'-amino benzalmalonate de di(2-éthylhexyle))-6-(4'-aminobenzoate de 2-éthylhexyle)-s-triazine,
la 2,4,6-tris-(4'-amino benzalmalonate de diisobutyle)-6-butoxy-s-triazine,
la 2,4,6-tris-(4'-amino benzalmalonate de diisobutyte)-6-(2-éthythexytamino)-s-triazine,
la 2,4-bis-(4'-aminobenzylidène camphre)-6-(2-éthylhexyl-amino)-s-triazine,
la 2,4-bis-(4'-aminobenzylidène camphre)-6-(4'-amino benzalmalonate de diisobutyle)-s-triazine,
la 2,4,6-tris(4'-amino benzalmalonate de diéthyle)-s-triazine,
la 2,4,6-tris(4'-amino benzalmalonate de diisopropyle)-s-triazine,
la 2,4,6-tris(4'-amino benzalmalonate de diméthyle)-s-triazine,
la 2,4,6-tris(α-cyano-4-aminocinnamate d'éthyle)-s-triazine.
la 2,4,6-tris[(3'-benzotriazol-2-yl-2'-hydroxy-5'-methyl) phenylamino]-s-triazine,
2,4,6-tris[(3'-benzotriazol-2-yl-2'-hydroxy-5'-ter-octyl) phénylamino]-s-triazine.

15. Composition selon la revendication 14, où le dérivé de 1,3,5-triazine est la 2,4,6-tris-(4'-amino benzalmalonate de diisobutyle)-s- triazine.

16. Composition selon l'une quelconque des revendications 1 à 15, où le ou les dérivés de 1,3,5-triazine sont présents à des concentrations allant de 0,05 à 15% et de préférence de 0,1 à 10 % en poids par rapport au poids total de ladite composition.

17. Composition selon l'une quelconque des revendications 1 à 16, contenant en plus au moins un filtre organique et/ou au moins un filtre minéral complémentaire, hydrosoluble, liposoluble ou insoluble dans les solvants cosmétiques couramment utilisés.

18. Composition selon la revendication 17, où les filtres organiques complémentaires sont choisis parmi les anthranilates ; les dérivés cinnamiques ; les dérivés de dibenzoylméthane ; les dérivés salicyliques, les dérivés du camphre ; les dérivés de la benzophénone ; les dérivés de β,β'-diphénylacrylate ; les dérivés de benzotriazole ; les dérivés de benzalmalonate ; les dérivés de benzimidazole ; les imadazolines ; les dérivés bis-benzoazolyle ; les dérivés de l'acide p-aminobenzoïque (PABA) ; les dérivés de méthylène bis-(hydroxyphényl benzotriazole) ; les dérivés de benzoxazole ; les polymères filtres et silicones filtres ; les dimères dérivés d'α-alkylstyrène ; les 4,4-diarylbutadiènes et leurs mélanges.

19. Composition selon la revendication 18, où les filtres organiques complémentaires sont choisis parmi :
Ethylhexyl Salicylate,
Ethylhexyl Methoxycinnamate
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethykhexyl)-imino-1,3,5-triazine
et leurs mélanges.

20. Composition selon la revendication 19, où les filtres complémentaires inorganiques sont choisis parmi des pigments ou des nanopigments d'oxydes métalliques, enrobés ou non.

21. Composition selon la revendication 20, où les filtres complémentaires inorganiques sont des nanopigments d'oxyde de titane, amorphe ou cristallisé, sous forme rutile et/ou anatase, de fer, de zinc, de zirconium ou de cérium

22. Composition selon l'une quelconque des revendications 17 à 21, où les filtres complémentaires sont présents dans des proportions allant de 0,1 à 20% en poids par rapport au poids total de la composition, et de préférence allant de 0,2 à 15% en poids par rapport au poids total de la composition.

23. Composition selon l'une quelconque des revendications 1 à 22, contenant en plus des adjuvants cosmétiques choisis parmi les corps gras, les solvants organiques autres que ceux utilisés spécifiquement dans le cadre de la présente invention, les émulsionnants, les épaississants ioniques ou non ioniques, les adoucissants, les antioxydants, les agents anti-radicaux libres, les opacifiants, les stabilisants, les émollients, les silicones, les α-hydroxyacides, les agents anti-mousse, les agents hydratants, les vitamines, les agents répulsifs contre les insectes, les parfums, les conservateurs, les tensioactifs, les anti-inflammatoires, les antagonistes de substance P, les charges, les polymères, les propulseurs, les agents alcalinisants ou acidifiants, les colorants.

24. Composition selon l'une quelconque des revendications 1 à 23, **caractérisée en ce qu'**il s'agit d'une composition protectrice de l'épiderme humain et qu'elle se présente sous forme de suspension ou de dispersion, sous forme de dispersion vésiculaire non ionique, sous forme d'émulsion, sous forme de pommade, de gel, de gel crème, de bâtonnet solide, de poudre, de lingettes, de mousse aérosol ou de spray.

25. Composition selon l'une quelconques des revendications 1 à 23, **caractérisée en ce qu'**il s'agit d'une composition de maquillage des cils, des sourcils, des ongles ou de la peau et qu'elle se présente sous forme solide ou pâteuse, anhydre ou aqueuse.

26. Composition selon l'une quelconque des revendications 1 à 23, **caractérisée en ce qu'**il s'agit d'une composition destinée à la protection des cheveux contre les rayons ultraviolets et qu'elle se présente sous forme de shampooing, de lotion, de gel, d'émulsion, de dispersion vésiculaire non ionique.

27. Utilisation d'une composition selon l'une quelconque des revendications précédentes pour la fabrication de compositions cosmétiques ou dermatologiques destinées à la protection de la peau et/ou des lèvres et/ou des phanères contre le rayonnement ultraviolet, en particulier le rayonnement solaire.

28. Utilisation d'au moins un mélange eutectique n-butylphthalimide/isopropylphthalimide tel que défini dans les revendications précédentes dans une composition cosmétique ou dermatologique contenant un dérivé de 1,3,5-triazine tel que défini dans les revendications précédentes comme solvant du dérivé de triazine dans la composition

29. Utilisation d'au moins un mélange eutectique n-butylphthalimide/isopropylphthalimide tel que défini dans les revendications précédentes dans une composition cosmétique ou dermatologique contenant un dérivé de 1,3,5-triazine tel que défini dans les revendications précédentes en vue d'améliorer le facteur de protection solaire, les propriétés cosmétiques et/ou la stabilité de cette composition.

## Claims

1. Photoprotective composition, **characterized in that** it comprises:
(i) at least one 1,3,5-triazine derivative, and
(ii) at least one eutectic mixture consisting of:
(a) n-butylphthalimide of structure (1) below: 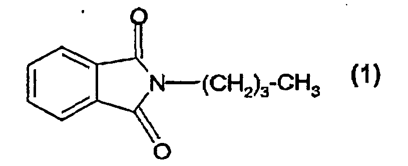
(b) isopropylphthalimide of structure (2) below: 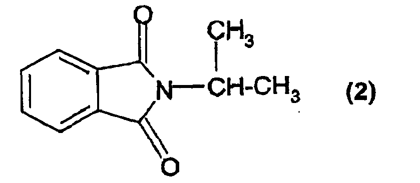
in an amount sufficient to allow it, by itself, to dissolve all of the triazine derivative.

2. Composition according to Claim 1, in which the said eutectic mixture contains from 60% to 75% by weight of n-butylphthalimide.

3. Composition according to Claim 1 or 2, in which the eutectic mixture is chosen from the following mixtures:
n-butylphthalimide/isopxopylphthalimide (60/40% by weight)
n-butylphthalimide/isopropylphthalimide (62/38% by weight)
n-butylphthalimide/isopropylphthalimide (65/35% by weight)
n-butylphthalimide/isopropylphthalimide (70/30% by weight)
n-butylphthalimide/isopropylphthalimide (75/25% by weight) .

4. Composition according to any one of Claims 1 to 3, in which the eutectic mixture is present in concentrations ranging from 0.1% to 50% by weight and more preferably from 1% to 30% by weight relative to the total weight of the composition.

5. Composition according to any one of Claims 1 to 4, in which the 1,3,5-triazine derivative corresponds to formula (I) below: 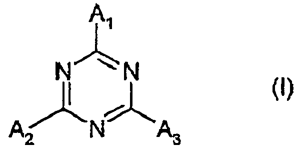 in which the radicals A₁, A₂ and A₃, which may be identical or different, are chosen from the groups of formulae (II) to (IX) below: 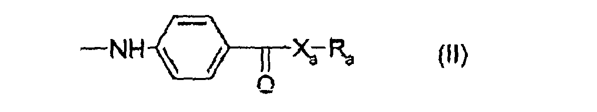 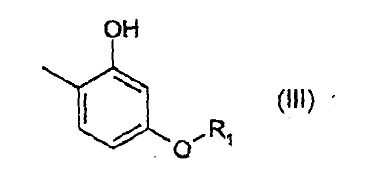 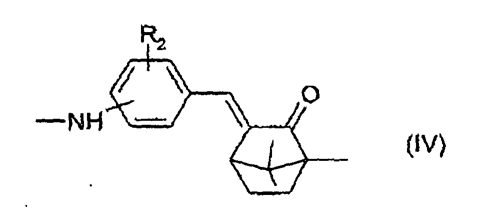 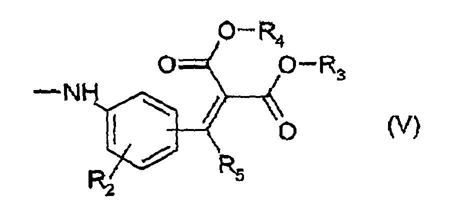 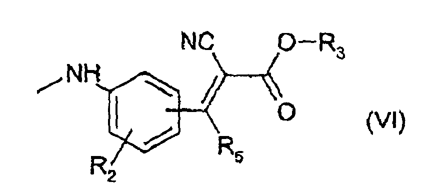 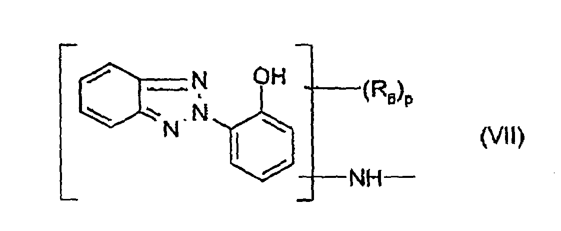 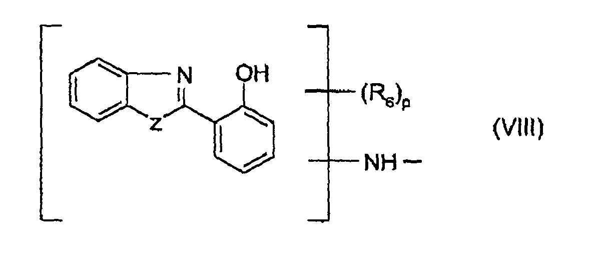 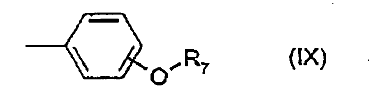 in which:
- Xₐ (each of the groups Xₐ may be identical or different) represents oxygen or -NH-;
- Rₐ (each of the groups Rₐ may be identical or different) is chosen from hydrogen; an alkali metal; an ammonium radical optionally substituted with one or more linear or branched C₁-C₁₈ alkyl or linear or branched C₁-C₁₈ hydroxyalkyl radicals; a linear or branched C₁-C₁₈ and preferably C₆-C₁₂ alkyl radical; a C₅₋C₁₂ cycloalkyl radical optionally substituted with one or more C₁-C₄ alkyl radicals; a polyoxyethylenated radical comprising from 1 to 6 ethylene oxide units, the terminal OH group of which is methylated; a radical of formula (X), (XI) or (XII) below: 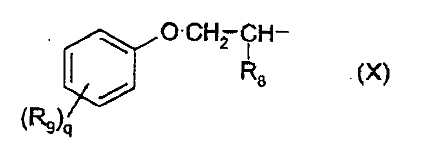 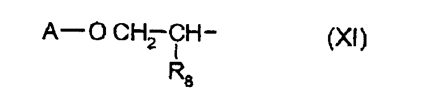 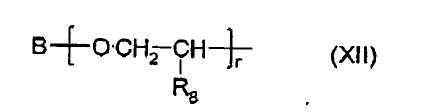
in which:
- R₈ is hydrogen or a methyl radical;
- R₉ is a C₁-C₉ alkyl radical;
- q is an integer equal to 0; 1; 2; 3;
- r is an integer equal to 1; 2; 3; 4; 5; 6; 7; 8; 9; 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₁ denotes a C₃-C₁₈ alkyl radical; a C₂-C₁₈ alkenyl radical; a residue of formula -CH₂-CH (OH) -CH₂-OT₁ in which T₁ is a hydrogen atom or a C₁-C₈ alkyl radical; a residue of formula (XIII) below: 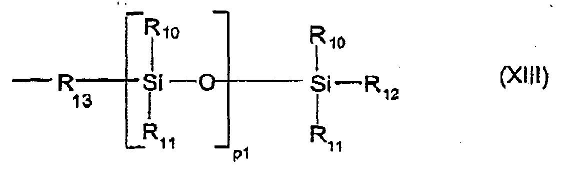
in which:
- R₁₃ denotes a covalent bond; a linear or branched C₁-C₄ alkyl radical or a radical of formula -Cₘ₁H₂ₘ₁-O- in which m₁ is an integer equal to 1; 2; 3; 4;
- p₁ is an integer equal to 0; 1; 2; 3; 4; 5;
- the radicals R₁₀, R₁₁ and R₁₂, which may be identical or different, denote a C₁-C₁₈ alkyl radical; a C₁-C₁₈ alkoxy radical or a radical of formula: 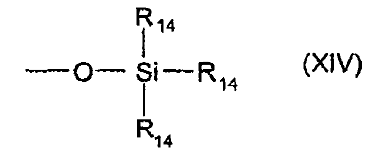
in which R₁₄ is a C₁-C₅ alkyl radical;
- R₂ denotes a hydrogen atom, a linear or branched C₁₋C₄ alkyl radical or a C₁-C₄ alkoxy radical;
- R₃ and R₄, which may be identical or different, denote a linear or branched C₁-C₂₀ alkyl radical;
- R₅ represents a hydrogen atom or a phenyl radical optionally substituted with a halogen or with a C₁-C₄ alkyl radical or with a C₁-C₄ alkoxy radical;
- R₆ is a linear or branched C₁-C₈ alkyl radical or a C₁-C₃ alkoxy radical, it being understood that, in the latter case, two adjacent radicals R₆ on the same aromatic nucleus can together form an alkylidenedioxy group in which the alkylidene group contains 1 or 2 carbon atoms, OH, NHCOCH₃ or NH₂,
- R₇ denotes a hydrogen atom, a C₁-C₁₀ alkyl radical, a radical of formula : - (CH₂CHR₅-O) n₁R₈ in which n₁ is a number from 1 to 16, or a radical of structure -CH₂-CH-(OH) -CH₂OT₁ with R₈ and T₁ having the same meaning as indicated above,
- Z represents oxygen, sulfur, -NH- or -NR₃- with R₃ representing a linear or branched C₁-C₂₀ alkyl radical;
- p is 0, 1, 2 or 3,
A₁ can also be a halogen, a radical -N (R₃)₂, the two radicals R₃ together possibly forming a ring of 4 or 5 carbon atoms, or a group -OR₃, R₃ having the same definition as above.

6. Composition according to Claim 5, in which the 1,3,5-triazine derivative corresponds to formula (I) in which A₁, A₂ and A₃ are of formula (II) and have all of the following characteristics:
- one of the groups Xₐ-Rₐ represents a radical-NH-Rₐ with Rₐ chosen from: a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
- the other two groups Xₐ-Rₐ represent a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

7. Composition according to Claim 5, in which the 1,3,5-triazine derivative corresponds to formula (I) in which A₁, A₂ and A₃ are of formula (II) and have all of the following characteristics:
- one or two groups Xₐ-Rₐ represent a radical -NH-Rₐ, with Rₐ chosen from: a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical;
the other or the other two group(s) Xₐ-Rₐ being a radical -O-Rₐ with Rₐ, which may be identical or different, chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (X), (XI) or (XII) above in which:
- B is a C₁-C₄ alkyl radical;
- R₉ is a methyl radical.

8. Composition according to Claim 7, in which the 1,3,5-triazine derivative is 2-[(p-(tert-butylamido)-anilino]-4,6-bis[(p-(2'-ethylhexyl-1'-oxycarbonyl)-anilino]-1,3,5-triazine of the following formula: 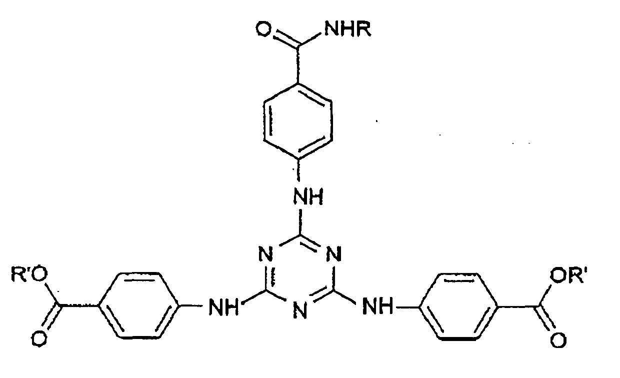 in which R' denotes a 2-ethylhexyl radical and R denotes a tert-butyl radical.

9. Composition according to Claim 5, in which the 1,3,5-triazine derivative corresponds to formula (I) in which A₁, A₂ and A₃ are of formula (II) and have all of the following characteristics:
- Xₐ are identical and represent oxygen;
- Rₐ, which may be identical or different, represent a C₆-C₁₂ alkyl radical or a polyoxyethylenated radical comprising from 1 to 6 ethylene oxide units, the terminal OH group of which is methylated.

10. Composition according to Claim 9, in which the 1,3,5-triazine derivative is 2,4,6-tris[p(2'-ethylhexyl-1'-oxycarbonyl)anilino]-1,3,5-triazine of the following formula: 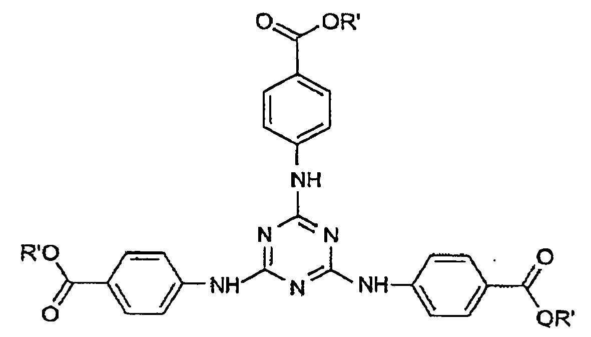 in which R' denotes a 2-ethylhexyl radical.

11. Composition according to Claim 5, in which the 1,3,5-triazine derivative corresponds to formula (I) in which A₁ and A₂ are of formula (III) and A₃ is of formula (IX) and have all of the following characteristics: R₁, which may be identical or different, denote a C₃-C₁₈ alkyl radical; a C₂-C₁₈ alkenyl radical or a residue of formula -CH₂-CH(OH)-CH₂₋OT₁ in which T₁ is a hydrogen atom or a C₁-C₈ alkyl radical; R₇ denotes a hydrogen atom or a C₁-C₁₀ alkyl radical.

12. Composition according to Claim 11, in which the 1,3,5-triazine derivative is 2,4-bis{[4-2-ethylhexyloxy)]-2-hydroxy]phenyl}-6-(4-methoxyphenyl)-1,3,5-triazine of the following formula: 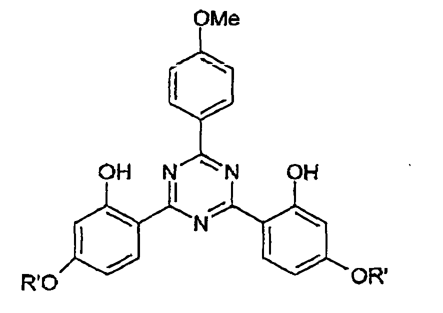 in which R' denotes a 2-ethylhexyl radical.

13. Composition according to Claim 5, in which the 1,3,5-triazine derivative corresponds to formula (I) in which A₁, A₂ and A₃ are of formulae (VII) to (XI).

14. Composition according to Claim 13, in which the 1,3,5-triazine derivative is chosen from:
2,4,6-tris(diisobutyl 4'-aminobenzalmalonate)-s-triazine,
2,4,6-tris(bis(2-ethylhexyl) 4'-aminobenzalmalonate)-s-triazine,
2, 4, 6-tris (bis (2-ethylhexyl) 4'-aminobenzalmalonate) -6-chloro-s-triazine,
2, 4, 6-tris (bis (2-ethylhexyl) 4'-aminobenzalmalonate)-6-(2-ethylhexyl 4'-aminobenzoate)-s-triazine,
2, 4, 6-tris (diisobutyl 4' -aminobenzalmalonate)-6-butoxy-s-triazine,
2,4,6-tris(diisobutyl 4'-aminobenzalmalonate)-6-(2-ethylhexylamino)-s-triazine,
2,4-bis(4'-aminobenzylidenecamphor)-6-(2-ethyl-hexylamino)-s-triazine,
2,4-bis(4'-aminobenzylidenecamphor)-6-(diisobutyl 4'-aminobenzalmalonate)-s-triazine,
2,4,6-tris(diethyl 4'-aminobenzalmalonate)-s-triazine,
2,4,6-tris(diisopropyl 4'-aminobenzalmalonate)-s-triazine,
2,4,6-tris(dimethyl 4'-aminobenzalmalonate)-s-triazine,
2,4,6-tris(ethyl α-cyano-4-aminocinnamate)-s-triazine,
2,4,6-tris[(3'-benzotriazol-2-yl-2'-hydroxy-5'-methyl)phenylamino]-s-triazine,
2,4,6-tris[(3'-benzotriazol-2-yl-2'-hydroxy-5'-tert-octyl)phenylamino]-s-triazine.

15. Composition according to Claim 14, in which the 1,3,5-triazine derivative is 2,4,6-tris(diisobutyl 4'-aminobenzalmalonate)-s-triazine.

16. Composition according to any one of Claims 1 to 15, in which the 1,3,5-triazine derivative(s) is (are) present in concentrations ranging from 0.05% to 15% and preferably from 0.1% to 10% by weight relative to the total weight of the said composition.

17. Composition according to any one of Claims 1 to 16, also containing at least one additional organic photoprotective agent and/or at least one additional mineral photoprotective agent, which is water-soluble, liposoluble or insoluble in the cosmetic solvents commonly used.

18. Composition according to Claim 17, in which the additional organic photoprotective agents are chosen from anthranilates; cinnamic derivatives; dibenzoylmethane derivatives; salicylic derivatives; camphor derivatives; benzophenone derivatives; β,β'-diphenyl-acrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxy-phenylbenzotriazole) derivatives; benzoxazole derivatives; screening polymers and screening silicones; dimers derived from α-alkylstyrene; 4,4-diarylbutadienes, and mixtures thereof.

19. Composition according to Claim 18, in which the additional organic photoprotective agents are chosen from:
Ethylhexyl salicylate,
Ethylhexyl methoxycinnamate,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
Methylenebis(benzotriazolyl)tetramethylbutylphenol,
Drometrizole trisiloxane,
Polysilicone-15,
1,1-Dicarboxy(2,2'-dimethylpropyl)-4,4-diphenyl-butadiene,
2,4-Bis[5-1(dimethylpropyl)benzoxazol-2-yl(4-phenyl)imino]-6-(2-ethylhexyl)imino-1,3,5-triazine,
and mixtures thereof.

20. Composition according to Claim 19, in which the additional mineral photoprotective agents are chosen from coated or uncoated metal oxide pigments or nanopigments.

21. Composition according to Claim 20, in which the additional mineral photoprotective agents are nanopigments of titanium oxide, which is amorphous or crystallized in rutile and/or anatase form, or of iron oxide, zinc oxide, zirconium oxide or cerium oxide.

22. Composition according to any one of Claims 17 to 21, in which the additional photoprotective agents are present in proportions ranging from 0.1% to 20% by weight relative to the total weight of the composition, and preferably ranging from 0.2% to 15% by weight relative to the total weight of the composition.

23. Composition according to any one of Claims 1 to 22, also containing cosmetic adjuvants chosen from fatty substances, organic solvents other than those specifically used in the context of the present invention, emulsifiers, ionic or nonionic thickeners, softeners, antioxidants, free-radical scavengers, opacifiers, stabilizers, emollients, silicones, α-hydroxy acids, antifoams, moisturizers, vitamins, insect repellents, fragrances, preserving agents, surfactants, anti-inflammatory agents, substance P antagonists, fillers, polymers, propellants, acidifying or basifying agents, and dyes.

24. Composition according to any one of Claims 1 to 23, **characterized in that** it is a composition for protecting the human epidermis and **in that** it is in the form of a suspension or a dispersion, in the form of a nonionic vesicular dispersion, in the form of an emulsion or in the form of an ointment, gel, cream-gel, solid stick, powder, wipes, aerosol mousse or spray.

25. Composition according to any one of Claims 1 to 23, **characterized in that** it is a makeup composition for the eyelashes, the eyebrows, the nails or the skin and **in that** it is in solid or pasty, anhydrous or aqueous form.

26. Composition according to any one of Claims 1 to 23, **characterized in that** it is a composition for protecting the hair against ultraviolet rays and **in that** it is in the form of a shampoo, a lotion, a gel, an emulsion or a nonionic vesicular dispersion.

27. Use of a composition according to any one of the preceding claims, for the manufacture of cosmetic or dermatological compositions for protecting the skin and/or the lips and/or the integuments against ultraviolet radiation, in particular sunlight.

28. Use of at least one n-butylphthalimide/isopropylphthalimide eutectic mixture as defined in the preceding claims in a cosmetic or dermatological composition containing a 1,3,5-triazine derivative as defined in the preceding claims, as solvent for the triazine derivative in the composition.

29. Use of at least one n-butylphthalimide/isopropylphthalimide eutectic mixture as defined in the preceding claims in a cosmetic or dermatological composition containing a 1,3,5-triazine derivative as defined in the preceding claims, in order to improve the sun protection factor, the cosmetic properties and/or the stability of this composition.

## Patentansprüche

1. Lichtschutzzusammensetzung, **dadurch gekennzeichnet, dass** sie enthält:
(i) mindestens ein 1,3,5-Triazinderivat und
(ii) mindestens ein eutektisches Gemisch, bestehend aus:
(a) dem *n*-Butylphthalimid der folgenden Struktur (1): 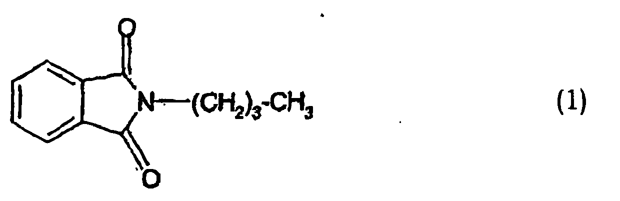
(b) dem Isopropylphthalimid der folgenden Struktur (2): 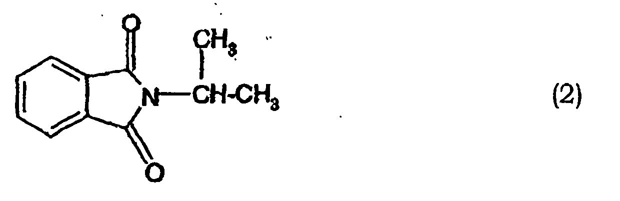
in einer Menge, die ausreichend ist, alleine die Gesamtmenge des Triazinderivats zu solubilisieren.

2. Zusammensetzung nach Anspruch 1, wobei das eutektische Gemisch 60 bis 75 Gew.-% *n*-Butylphthalimid enthält.

3. Zusammensetzung nach Anspruch 1 oder 2, wobei das eutektische Gemisch unter den folgenden Gemischen ausgewählt ist:
*n*-Butylphthalimid/Isopropylphthalimid (60/40 Gew.-%)
*n*-Butylphthalimid/Isopropylphthalimid (62/38 Gew.-%)
*n*-Butylphthalimid/Isopropylphthalimid (65/35 Gew,-%)
*n*-B-utylphthalimid/Isopropylphthalimid (70/30 Gew.-%)
*n*-Butylphthalimid/Isopropylphthalimid (75/25 Gew.-%).

4. Zusammensetzung nach einem der Ansprüche 1 bis 3, wobei das eutektische Gemisch in Konzentrationen von 0,1 bis 50 Gew.-% und vorzugsweise 1 bis 30 Gew.-%, bezogen auf das Gesamtgewicht der Zusammensetzung, enthalten ist.

5. Zusammensetzung nach einem der Ansprüche 1 bis 4, wobei das 1,3,5-Triazinderivat der folgenden Formel (I) enstpricht: 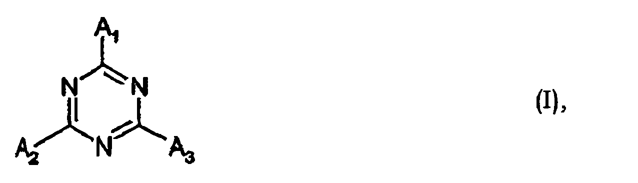 worin die Gruppen A₁, A₂ und A₃, die gleich oder verschieden sind, unter den Gruppen der folgenden Formel (II) bis (IX) ausgewählt sind: 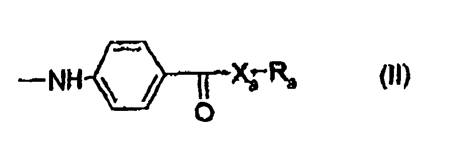 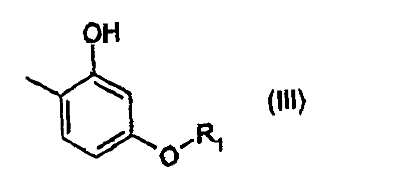 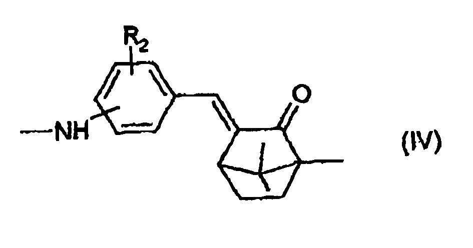 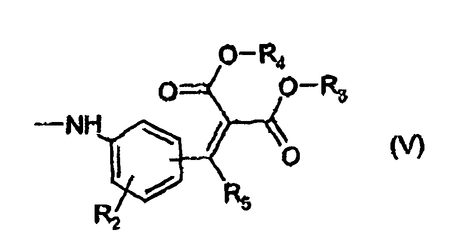 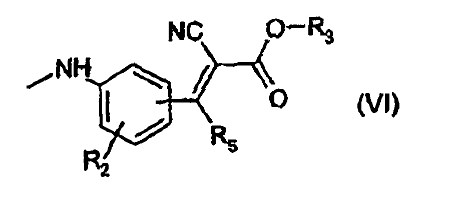 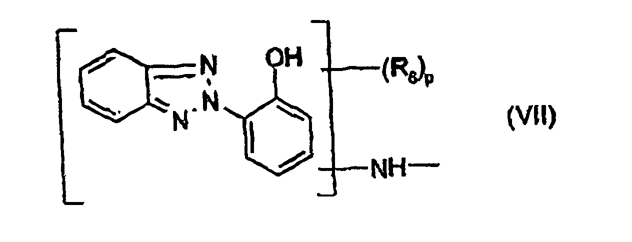 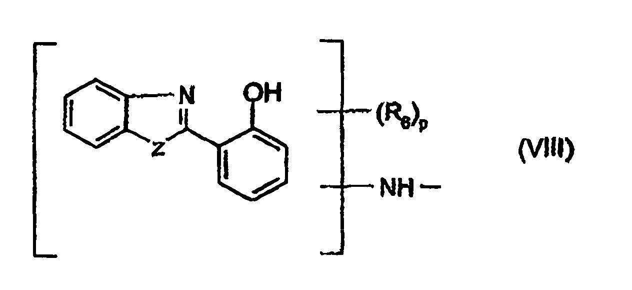 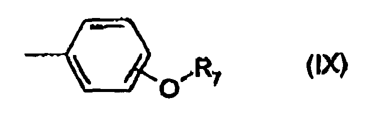 worin bedeuten:
- Xₐ (wobei die Gruppen Xₐ gleich oder verschieden sein können) Sauerstoff oder -NH-;
- Rₐ (wobei die Gruppen Rₐ gleich oder verschieden sein können) Wasserstoff; ein Alkalimetall, eine Ammoniumgruppe, die gegebenenfalls mit einer oder mehreren geradkettigen oder verzweigten C₁-₁₈-Alkylgruppen oder geradkettigen oder verzweigten C₁₋₁₈-Hydroxyalkylgruppen substituiert sein kann; eine geradkettige oder verzweigte C₁₋₁₈-Alkylgruppe und vorzugsweise C₆₋₁₂-Alkylgruppe; eine C₅₋₁₂-Cycloalkylglruppe, die gegebenenfalls mit einer oder mehreren C₁₋₄-Alkylgruppen substituiert ist; eine polyethoxylierte Gruppe mit 1 bis 6 Ethylenoxideinheiten, deren endständige OH-Gruppe methyliert ist; eine Gruppe der folgenden Formel (X), (XI) oder (XII) : 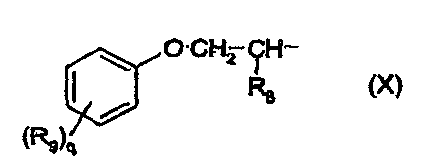 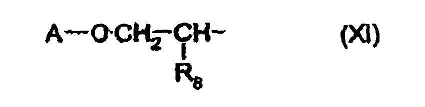 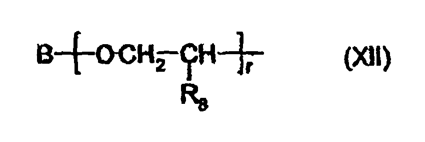
worin bedeuten:
- R₈ Wasserstoff oder Methyl;
- R₉ C₁₋₉-Alkyl,
- q 0; 1; 2; 3;
- r eine ganze Zahl 1; 2; 3; 4; 5; 6; 7; 8; 9; 10;
- A C₄₋₈-Alkyl oder C₅₋₈-Cycloalkyl;
- B ist ausgewählt unter: einer geradkettigen oder verzweigten C₁₋₈-Alkylgruppe; einer C₅₋₈-Cycloalkylgruppe; einer Arylgruppe, die gegebenenfalls mit einer oder mehreren C₁₋₄-Alkylgruppen substituiert ist;
- R₁ C₃₋₁₈-Alkyl; C₂₋₁₈-Alkenyl; einen Rest der Formel -CH₂-CH(OH)-CH₂-OT₁, wobei T₁ Wasserstoff oder C₁₋₈-Alkyl bedeutet; einen Rest der folgenden Formel (XIII): 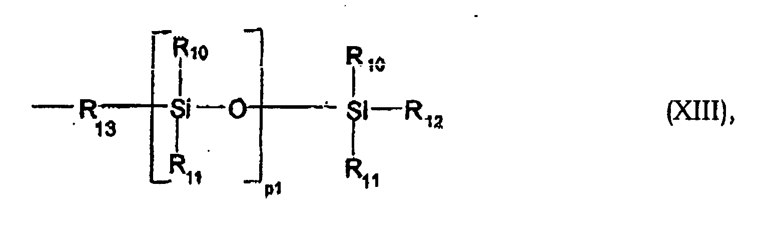
worin bedeuten:
- R₁₃ eine kovalente Bindung; eine geradkettige oder verzweigte C₁₋₄-Alkylgruppe oder eine Gruppe der Formel -Cₘ₁H₂ₘ₁O-, wobei m₁ eine ganze Zahl 1; 2; 3; 4; bedeutet;
- p₁ 0 oder eine ganze Zahl 1; 2; 3; 4; 5;
- die Gruppen R₁₀, R₁₁ und R₁₂, die gleich oder verschieden sind, C₁-₁₈-Alkyl, C₁₋₁₈-Alkoxy oder eine Gruppe der Formel: 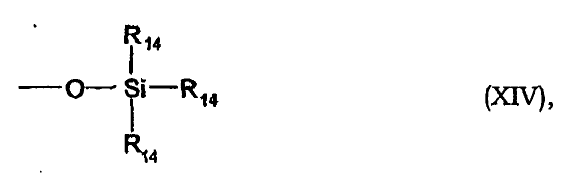
worin R₁₄ C₁₋₅-Alkyl bedeutet;
- R₂ ein Wasserstoffatom, eine geradkettige oder verzweigte C₁₋₄-Alkylgruppe oder eine C₁₋₄-Alkoxygruppe;
- R₃ und R₄, die gleich oder verschieden sind, eine geradkettige oder verzweigte C₁₋₂₀-Alkylgruppe;
- R₅ ein Wasserstoffatom oder eine Phenylgruppe, die gegebenenfalls mit einem Halogen oder einer C₁₋₄-Alkylgruppe oder einer C₁₋₄-Alkoxygruppe substituiert ist;
- R₆ eine geradkettige oder verzweigte C₁₋₈-Alkylgruppe, C₁₋₃-Alkoxy, mit der Maßgabe, dass in dem zuletzt genannten Fall zwei angrenzende Gruppen R₆ an einem aromatischen Ring gemeinsam eine Alkylidendioxygruppe bilden können, worin die Alkylidengruppe 1 bis 2 Kohlenstoffatome enthält, OH, NHCOCH₃ oder NH₂,
- R₇ ein Wasserstoffatom, C₁₋₁₀-Alkyl, eine Gruppe der Formel -(CH₂CHR₅-O)ₙ₁R₈, worin n₁ eine Zahl von 1 bis 16 bedeutet, oder eine Gruppe der Struktur -CH₂-CH-(OH)-CH₂OT₁, wobei R₈ und die T₁ die oben abgegebenen Bedeutungen aufweisen,
- Z Sauerstoff, Schwefel, -NH- oder -NR₃-, wobei R₃ eine geradkettige oder verzweigte C₁₋₂₀-Alkylgruppe bedeutet;
- p 0, 1, 2 oder 3,
A₁ kann auch ein Halogen bedeuten, eine Gruppe -N(R₃)₂, wobei zwei Gruppen R₃ gemeinsam einen Ring mit 4 oder 5 Kohlenstoffatomen bilden können, eine Gruppe -OR₃, wobei R₃ die oben angegebenen Bedeutungen aufweist.

6. Zusammensetzung nach Anspruch 5, wobei das 1,3,5-Triazinderivat der Formel (I) entspricht, worin die Gruppen A₁, A₂ und A₃ die Formel (II) aufweisen und alle folgenden Eigenschaften haben:
- eine der Gruppen Xₐ-Rₐ bedeutet die Gruppe -NH-Rₐ, wobei Rₐ ausgewählt ist unter: einer C₅₋₁₂-Cycloalkylgruppe, die gegebenenfalls mit einer oder mehreren C₁₋₄-Alkylgruppen substituiert ist; einer Gruppe der Formel (X), (XI) oder (XII), worin bedeuten:
- B eine C₁₋₄-Alkylgruppe;
- R₉ die Methylgruppe;
- die beiden anderen Gruppen XₐRₐ bedeuten die Gruppe -O-Rₐ, wobei die Gruppen Rₐ, die gleich oder verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (X), (XI) oder (XII), worin bedeuten:
- B eine C₁₋₄-Alkylgruppe;
- R₉ die Methylgruppe.

7. Zusammensetzung nach Anspruch 5, wobei das 1,3,5-Triazinderivat der Formel (I) entspricht, worin die Gruppen A₁, A₂ und A₃ die Formel (II) aufweisen und alle folgenden Eigenschaften besitzen:
- eine oder zwei Gruppen Xₐ-Rₐ bedeuten die Gruppe NH - Rₐ, wobei Rₐ ausgewählt ist unter: einer geradkettigen oder verzweigten C₁₋₁₈-Alkylgruppe; einer C₅₋₁₂-Cycloalkylgruppe, die gegebenenfalls mit einer oder mehreren C₁₋₄-Akylgruppen substituiert ist; einer oben genannten Gruppe der Formel (X), (XI) oder (XII), worin bedeuten:
- B eine C₁₋₄-Alkylgruppe;
- R₉ die Methylgruppe;
wobei die andere Gruppe Xₐ-Rₐ oder die beiden anderen Gruppen Xₐ-Rₐ die Gruppe -O-Rₐ bedeuten, wobei die Gruppen Rₐ, die gleich oder verschieden sind, ausgewählt sind unter: Wasserstoff; einem Alkalimetall, einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der Formel (X), (XI) oder (XII), worin bedeuten:
- B eine C₁₋₄-Alkylgruppe;
- R₉ die Methylgruppe.

8. Zusammensetzung nach Anspruch 7, wobei das 1,3,5-Triazinderivat das 2-[(p-(*t*-Butylamido)anilino]-4,6-bis[(p-(2'ethylhexyl-1'- oxycarbonyl)anilino]-1,3,5-triazin der folgenden Formel ist: 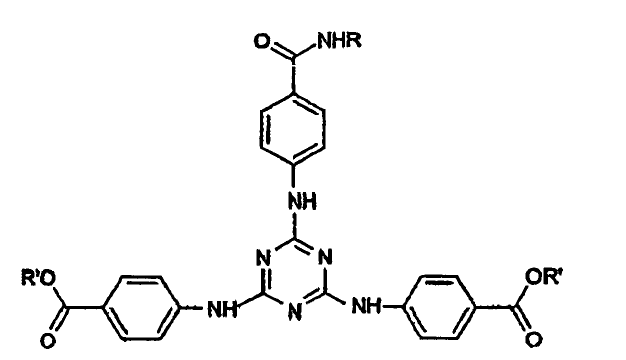 worin R' eine 2-Ethylhexylgruppe und R eine *t*-Butylgruppe bedeutet.

9. Zusammensetzung nach Anspruch 5, wobei das 1,3,5-Triazinderivat der Formel (I) entspricht, worin die Gruppen A₁, A₂ und A₃ die Formel (II) aufweisen und alle folgenden Eigenschaften haben:
- die Gruppen Xₐ sind identisch und bedeuten Sauerstoff; .
- die Gruppen Rₐ, die gleich oder verschieden sind, bedeuten eine C₆₋₁₂-Alkylgruppe oder eine polyethoxylierte Gruppe mit 1 bis 6 Ethylenoxideinheiten, deren endständige OH-Gruppe methyliert ist.

10. Zusammensetzung nach Anspruch 9, wobei das 1,3,5-Triazinderivat das 2,4,6-Tris[p-(2'-ethylhexyl-1'-oxycarbonyl)anilino]-1,3,5-triazin der folgenden Formel ist: 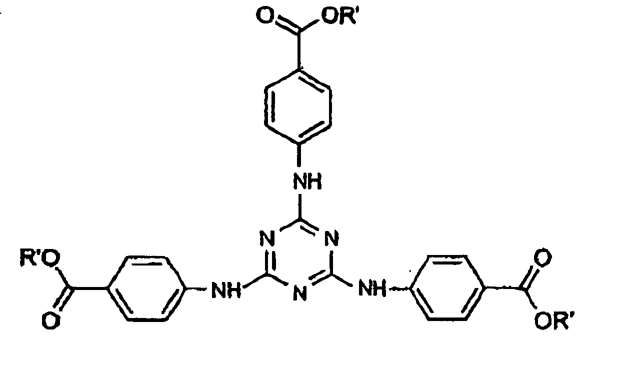 worin R' eine 2-Ethylhexylgruppe bedeutet,

11. Zusammensetzung nach Anspruch 5, wobei das 1,3,5-Triazinderivat der Formel (I) entspricht, worin die Gruppen A₁ und A₂ die Formel (III) aufweisen und A₃ die Formel (IX) besitzt, und alle folgenden Eigenschaften aufweist: die Gruppen R₁, die gleich oder verschieden sind, bedeuten C₃₋₁₈-Alkyl; C₂₋₁₈-Alkenyl oder einen Rest der Formel -CH₂-CH(OH)-CH₂-OT₁, wobei T₁ ein Wasserstoffatom oder eine C₁₋₈-Alkylgruppe bedeutet; R₇ bedeutet ein Wasserstoffatom; eine C₁₋₁₀-Alkylgruppe.

12. Zusammensetzung nach Anspruch 11, wobei das 1,3,5-Triazinderivat das 2,4-Bis{[4-(2-ethylhexyloxy)]-2-hydroxy]-phenyl}-6-(4-methoxyphenyl)-1,3,5-triazin der folgenden Formel ist: 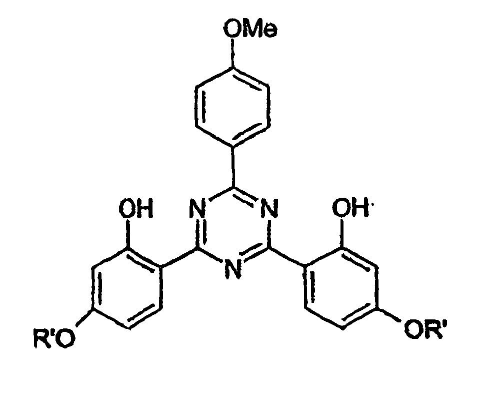 worin R' 2-Ethylhexyl bedeutet.

13. Zusammensetzung nach Anspruch 5, wobei das 1,3,5-Triazinderivat der Formel (I) entspricht, worin die Gruppen A₁, A₂ und A₃ die Formel (VII) bis (XI) aufweisen.

14. Zusammensetzung nach Anspruch 13, wobei das 1,3,5-Triazinderivat ausgewählt ist unter:
- 2,4,6-Tris(diisobutyl-4'-amino-benzalmalonat)-s-triazin,
- 2,4,6-Tris(di(2-ethylhexyl)-4'-amino-benzalmalonat)-s-triazin,
- 2,4,6-Tris(di(2-ethylhexyl)-4'-amino-benzalmalonat)-6-chlor-s-triazin,
- 2,4,6-Tris(di-(2-ethylhexyl)-4'amino-benzalmalonat)-6-(2-ethylhexyl-4'aminobenzoat)-s-triazin
- 2,4,6-Tris(diisobutyl-4'amino-benzalmalonat)-6-butoxy-s-triazin,
- 2,4,6-Tris(diisobutyl-4'-aminobenzalmalonat)-6-(2-ethylhexylamino)-2-triazin,
- 2,4-Bis(4'-aminobenzylidencampher)-6-(2-ethylhexylamino)-s-triazin,
- 2,4-Bis(4'-aminobenzylidencampher)-6-(diisobutyl-4'-amino-benzalmalonat)-s-triazin,
- 2,4,6-Tris(diethyl-4'-aminobenzalmalonat)-s-triazin,
- 2,4,6-Tris(diisopropyl-4'-aminobenzalmalonat)-s-triazin,
- 2,4,6-Tris(dimethyl-4'-aminobenzalmalonat)-s-triazin,
- 2,4,6-Tris(ethyl-α-cyano-4-aminocinnamat)-s-triazin,
- 2,4,6-Tris[(3'-benzotriazol-2-yl-2'-hydroxy-5'-methyl)phenylamino]-s-triazin,
- 2,4,6- Tris[(3'-benzotriazol-2-yl-2' -hydroxy-5'- t-octyl)phenylamino]-s-triazin.

15. Zusammensetzung nach Anspruch 14, wobei das 1,3,5-Triazinderivat das 2,4,6-Tris(diisobutyl-4'-aminobenzalmalonat)-s-triazin ist.

16. Zusammensetzung nach einem der Ansprüche 1 bis 15, wobei das 1,3,5-Triazinderivat oder die 1,3,5-Triazinderivate in Konzentrationen von 0,05 bis 15 % und vorzugsweise 0,1 bis 10 Gew.-%, bezogen auf das Gesamtgewicht der Zusammensetzung, enthalten sind.

17. Zusammensetzung nach einem der Ansprüche 1 bis 16, die ferner mindestens ein zusätzliches, wasserlöslicher fettlösliches oder in gewöhnlich verwendeten kosmetischen Lösungsmitteln unlösliches anorganisches Filter und/oder organisches Filter enthält.

18. Zusammensetzung nach Anspruch 17, wobei die zusätzlichen organischen Filter unter den Anthranilaten; Zimtsäurederivaten; Dibenzoylmethanderivaten; Salicylsäurederviaten, Campherderivaten; Benzophenonderivaten; β,β-Diphenylacrylatderivaten; Benzotriazolderivaten; Benzalmalonatderivaten; Benzimidazolderivaten; Imidazolinen; Bis-benzoazolyldrivaten; p-Aminobenzoesäurederivaten (PABA); Methylen-bis(hydroxyphenylbenzotriazol)-derivaten; Benzoxazolderivaten; polymeren Filtern und Siliconfiltern; von α-Alkylstyrol abgeleiteten Dimeren; 4,4-Diarylbutadienen und deren Gemischen ausgewählt sind.

19. Zusammensetzung nach Anspruch 18, wobei die zusätzlichen organischen Filter ausgewählt sind unter:
Ethylhexyl Salicylate,
Ethylhexyl Methoxycinnamate
Octocrylene,
Phenylbenzimidazole Sulfonic Acid,
Benzophenone-3,
Benzophenone-4
Benzophenone-5,
N-Hexyl-2-(4-diethylamino-2-hydroxybenzoyl)-benzoat,
4-Methylbenzylidene camphor,
Terephthalylidene Dicamphor Sulfonic Acid,
Disodium Phenyl Dibenzimidazole Tetra-sulfonate,
Methylene bis-Benzotriazolyl Tetramethylbuylphenol,
Drometrizole Trisiloxane,
Polysilicone-15,
1,1-Dicarboxy-(2,2'-dimethylpropyl)-4,4-diphenylbutadien,
2,4-Bis[5,1(dimethylpropyl)benzoxazol-2-yl-(4-phenyl)-imino]-6-(2-ethylhexyl)-imino-1,3,5-triazin,
und deren Gemischen.

20. Zusammensetzung nach Anspruch 19, wobei die ergänzenden organischen Filter unter den Pigmenten oder Nanopigmenten von Metalloxiden, die umhüllt oder nicht umhüllt sind, ausgewählt sind.

21. Zusammensetzung nach Anspruch 20, wobei die ergänzenden anorganischen Filter Nanopigmente von amorphem oder in Form von Rutil und/oder Anatas kristallisiertem Titanoxid, Eisenoxid, Zinkoxid, Zirconiumoxid oder Ceroxid sind.

22. Zusammensetzung nach einem der Ansprüche 17 bis 21, wobei die ergänzenden Filter in Mengenanteilen von 0,1 bis 20 Gew.-%, bezogen auf das Gesamtgewicht der Zusammensetzung, und vorzugsweise 0,2 bis 15 Gew.-%, bezogen auf das Gesamtgewicht der Zusammensetzung, enthalten sind.

23. Zusammensetzung nach einem der Ansprüche 1 bis 22, die ferner kosmetische Zusatzstoffe enthält, die unter den Fettsubstanzen, organischen Lösungsmitteln, die von den speziell im Rahmen der vorliegenden Erfindung verwendeten Lösungsmitteln verschieden sind, Emulgatoren, ionischen oder nichtionischen Verdickungsmitteln, reizlindernden Stoffen, Antioxidantien, Radikalfänger für freie Radiale, Trübungsmitteln, Stabilisatoren, Emollientien, Siliconen, α-Hydroxysäuren, Schaumverhütungsmitteln, Hydratisierungsmitteln, Vitaminen, insektenabwehrenden Wirkstoffen, Parfums, Konservierungsmitteln, grenzflächenaktiven Stoffen, entzündungshemmenden Wirkstoffen, Substanz P-Antagonisten, Füllstoffen, Polymeren, Treibmitteln, Alkalisierungsmitteln, Ansäuerungsmitteln und Farbmitteln ausgewählt sind.

24. Zusammensetzung nach einem der Ansprüche 1 bis 23, **dadurch gekennzeichnet, dass** es sich um eine Zusammensetzung zum Schutz der menschlichen Epidermis handelt und **dadurch**, dass sie als Suspension oder Dispersion, in Form einer nichtionischen Vesikeldispersion, als Emulsion, Pomade, Gel, Gelcreme, fester Stift, Pulver, in Form eines getränkten Tuches, als Aerosolschaum oder Spray vorliegt.

25. Zusammensetzung nach einem der Ansprüche 1 bis 23, **dadurch gekennzeichnet, dass** es sich um eine Zusammensetzung zum Schminken der Wimpern, der Augenbrauen, der Nägel oder der Haut handelt und **dadurch**, dass sie in fester oder pastöser, wasserfreier oder wässriger Form vorliegt.

26. Zusammensetzung nach einem der Ansprüche 1 bis 23, **dadurch gekennzeichnet, dass** es sich um eine Zusammensetzung handelt, die zum Schutz der Haare gegen UV-Strahlung vorgesehen ist, und **dadurch**, dass sie als Haarwaschmittel, Lotion, Gel, Emulsion, nichtionische Vesikeldispersion vorliegt.

27. Verwendung einer Zusammensetzung nach einem der vorhergehenden Ansprüche zur Herstellung von kosmetischen oder dermatologischen Zusammensetzungen, die für den Schutz der Haut und/oder Lippen und/oder der Hautanhangsgebilde gegen UV-Strahlung und besonders gegen Sonnenlicht vorgesehen sind.

28. Verwendung mindestens eines eutektischen Gemisches von *n*-Butylphthalimid/Isopropylphthalimid nach einem der vorhergehenden Ansprüche in einer kosmetischen oder dermatologisehen Zusammensetzung, die ein 1,3,5-Triazinderivat enthält, nach einem der vorhergehenden Ansprüche, als Lösungsmittel für das Triazinderivat in der Zusammensetzung.

29. Verwendung mindestens eines eutektischen Gemisches von *n*-Butylphthalimid/Isopropylphthalimid nach einem der vorhergehenden Ansprüche in einer kosmetischen oder dermatologischen Zusammensetzung, die ein 1,3,5-Triazinderivat nach einem der vorhergehenden Ansprüche enthält, um den Sonnenschutzfaktor, die kosmetischen Eigenschaften und/oder die Stabilität der Zusammensetzung zu verbessern.
